# EUROPÄISCHE PATENTSCHRIFT

(11) **EP 2 367 583 B1**
(45) Veröffentlichungstag und Bekanntmachung des Hinweises auf die Patenterteilung: **13.04.2016**
(21) Anmeldenummer: 09765036.0
(22) Anmeldetag: 03.12.2009
(51) Int. Cl.: A61M 5/30

(54) **NADELLOSER EINMALINJEKTOR MIT EINEM BIEGEELASTISCHEN GEHÄUSE**
NEEDLELESS SINGLE-USE INJECTOR HAVING A FLEXURALLY ELASTIC HOUSING
INJECTEUR SANS AIGUILLE À USAGE UNIQUE DOTÉ D'UN BOÎTIER ÉLASTIQUE EN FLEXION

(30) Priorität: 18.12.2008 DE 102008063517
(43) Veröffentlichungstag der Anmeldung: 28.09.2011
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2009/008613
(87) Internationale Veröffentlichungsnummer: WO 2010/069469

(56) Entgegenhaltungen:
- EP-A1- 0 518 416
- DE-A1-102007 008 369
- GB-A- 805 184
- US-A- 3 094 989
- US-A- 3 557 784

## Beschreibung

Die Erfindung betrifft einen nadellosen Einmalinjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet sind, wobei der Kolbenbetätigungsstempel zwischen dem Federenergiespeicher und dem Kolben der Zylinder-Kolben-Einheit positioniert ist, wobei der Federenergiespeicher mindestens ein vorgespanntes Federelement umfasst, wobei der federbelastete Kolbenbetätigungsstempel am Gehäuse lösbar abgestützt ist.

Aus der DE 10 2007 031 630 A1 ist u.a. ein derartiger Injektor bekannt. Mit Ausnahme der mechanischen Feder des Federenergiespeichers sind nahezu alle Bauteile des Injektors aufwendig aus Kunststoffen durch Spritzgießen gefertigt. Mechanisch hochbelastete Bauteile sind zusätzlich glasfaserverstärkt ausgeführt.

Die DE 10 2007 008 369 A1 offenbart einen Einweginjektor mit einem Gehäuse, in dem oder an dem - jeweils zumindest bereichsweise - ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst und wobei zumindest ein Teil des Kolbenbetätigungsstempels zwischen dem Federenergiespeicher und dem Kolben der Zylinder-Kolben-Einheit positioniert ist. Der federbelastete Kolbenbetätigungsstempel weist mindestens einen Zugstab auf, der im Bereich seines hinteren Endes mindestens eine Abstützfläche hat. An der oder den Abstützflächen liegen am Gehäuse abgestützte Sperrelemente an, deren sperrende Lage durch ein in einer Sperrstellung positioniertes Auslöseelement gesichert ist und dass das Auslöseelement eine Lösestellung hat, die ein Freigeben der Sperrelemente bewirkt.

Die EP 0 518 416 A1 offenbart eine Injektionsvorrichtung, umfassend eine Glasampulle mit einer Injektionskanüle und einem Kolben, der durch eine vorgespannte Feder langsam in die Glasampulle eingedrückt wird, um die in der Glasampulle befindliche Injektionsflüssigkeit herauszudrücken.

Der vorliegenden Erfindung liegt daher die Problemstellung zugrunde, einen modular aufgebauten Einmalinjektor zu entwickeln, der bei geringer Baugröße nur wenige Bauteile aufweist und bei einfacher Handhabung sowie einer kostengünstigen Herstellung eine sichere Lagerung und Funktion gewährleistet.

Diese Problemstellung wird mit den Merkmalen des Hauptanspruchs gelöst. Dazu besteht das Gehäuse des Injektors aus einem dünnwandigen Blechteil. Das Blechteil weist mindestens zwei Schenkel auf. Jeder Schenkel weist an seinem freien Ende - als Aufnahme des Zylinders der Zylinder-Kolben-Einheit - jeweils ein abgewinkeltes Halteelement oder eine Ausnehmung auf. Die Schenkel sind elastische Biegebalken, die im mittleren Bereich zur Ausbildung eines Abstützabschnitts für den Kolbenbetätigungsstempel jeweils z- oder s-förmig abgebogen sind. Die zwischen einem einzelnen Abstützabschnitt und dem Kolbenbetätigungsstempel gelegene Kontaktzone stellt ein den jeweiligen Schenkel nach außen drängendes Keilgetriebepaar dar. Die Auslöseeinheit umfasst mindestens ein auf dem Blechteil gleitfähig angeordnetes Auslöseelement, wobei die Abstützabschnitte bzw. die Anlageabschnitte nach außen in Längsnuten springen.

Mit der Erfindung wird hier beispielsweise ein nadelfreier Einmalinjektor vorgestellt, dessen Kolbenbetätigungsstempel bei einem Auslösevorgang des Einmalinjektors freigegeben wird. Dazu wird zum Vorspannen und Halten des Federenergiespeichers der Kolbenbetätigungsstempel über gehäuseseitige Schenkel formschlüssig fixiert. Die Schenkel werden zumindest bereichsweise von einem Auslöseelement umgeben und bis zum Gebrauch des Einmalinjektors in einer Sperrposition lösbar gehalten. Zum Auslösen des Injektors werden die zumindest bereichsweise biegeelastischen Schenkel quer zur Auslöserichtung freigegeben, so dass sich der Kolbenbetätigungsstempel - unter der Wirkung des Federenergiespeichers - zumindest annähernd parallel zur Mittellinie des Einmalinjektor, bewegen kann, um die im Zylinder der Zylinder-Kolben-Einheit vorhandene Injektionslösung über mindestens eine Düse auszustoßen.

Das Gehäuse ist dabei ein einfaches, dünnwandiges Blechteil, in der Regel sogar nur ein Blechstreifen, der die mechanische oder pneumatische Feder des Federenergiespeichers zusammen mit einem Kolbenbetätigungsstempel und einer Zylinder-Kolben-Einheit unter Zusammenwirkung mit dem Auslöseelement lagert. Der gestanzte oder geschnittene, mehrfach gebogene Blechstreifen ist außerordentlich kostengünstig aus einem Eisenwerkstoff oder einem Buntmetall herstellbar. Ideal sind Werkstoffe, die eine hohe Elastizitätsgrenze, eine hohe Zugfestigkeit und ein hohes Streckgrenzenverhältnis aufweisen.

Weitere Einzelheiten der Erfindung ergeben sich aus den Unteransprüchen und den nachfolgenden Beschreibungen schematisch dargestellter Ausführungsbeispiele.
- Figur 1:: Einmalinjektor mit zwei Biegeschenkeln;
- Figur 2:: wie Figur 1, jedoch um 90 Winkelgrade geschwenkt;
- Figur 3:: Querschnitt zu Figur 2;
- Figur 4:: Längsschnitt des Blechstreifens;
- Figur 5:: oberer Bereich des Blechstreifens;
- Figur 6:: Einmalinjektor bei einem Montagezwischenschritt;
- Figur 7:: oberer Gehäusebereich während der Montage;
- Figur 8:: Querschnitt zu Figur 7;
- Figur 9:: wie Figur 1, jedoch entsichert und betätigt;
- Figur 10:: Einmalinjektor, vereinfachte Ausführung u.a. mit steinförmigem Stempel bei einem Montagezwischenschritt;
- Figur 11:: Einmalinjektor, handelsfertig;
- Figur 12:: Querschnitt zu Figur 10;
- Figur 13:: wie Figur 11, jedoch entsichert und betätigt;
- Figur 14:: Vergrößerung zu Figur 10;
- Figur 15:: Vergrößerung zu Figur 11;
- Figur 16:: Vergrößerung zu Figur 13;
- Figur 17:: Vergrößerter Ausschnitt zu Figur 1;
- Figur 18:: vergrößerte Seitenteilansicht zu Figur 13;
- Figur 19:: vergrößerte Seitenteilansicht des Blechstreifens aus Figur 10;
- Figur 20:: Einmalinjektor mit zwei mehrfach gebogenen Schenkeln;
- Figur 21:: wie Figur 20, jedoch entsichert und betätigt.

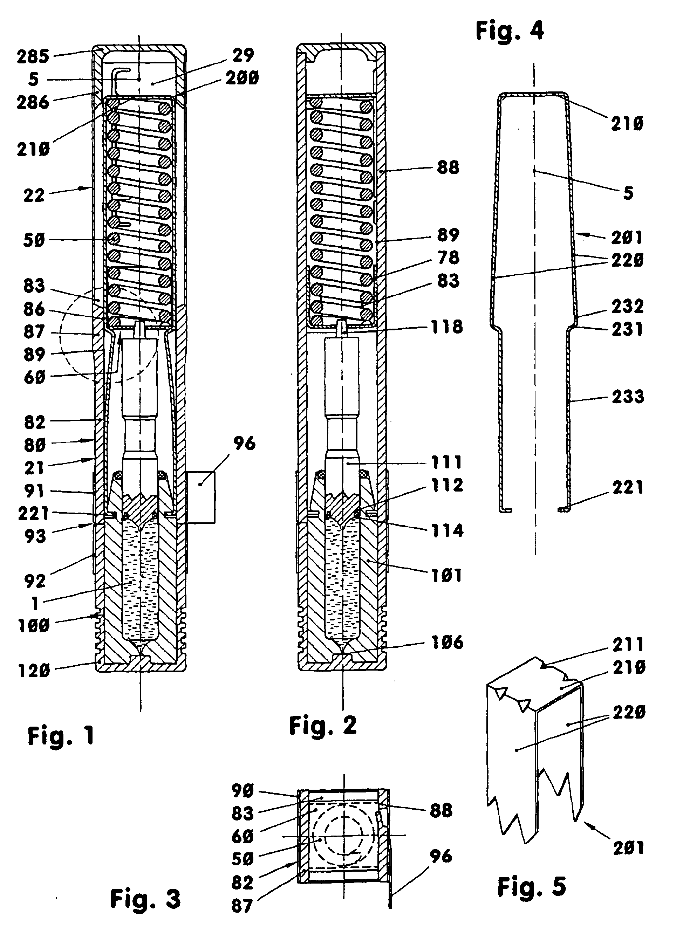

Die Figur 1 zeigt einen Einmal- bzw. Einweginjektor mit einem dauergeladenen Federenergiespeicher. Der Einweginjektor besteht aus einem von einem Auslöseelement (82) und einer Schutzkappe (120) umgebenen Gehäuse (200), einer z.B. mit einer Injektionslösung vorbefüllten Zylinder-Kolben-Einheit (100), einem Kolbenbetätigungsstempel (60) und einer Schraubendruckfeder (50) als Federenergiespeicher. Die Zylinder-Kolben-Einheit (100) sitzt dabei größtenteils in der Schutzkappe (120).

Das Gehäuse (200) ist ein zu einem "U" gebogener Blechstreifen (201). Der abgewickelte, z.B. 18 Millimeter breite, Blechstreifen (201) ist ca. 240 Millimeter lang. Der ggf. aus Federstahl gefertigte Blechstreifen (201) hat eine Wandstärke von z.B. 0,5 Millimeter. Der gebogene Blechstreifen (201) besteht aus einer zentralen Stirnplatte (210) und zwei davon zumindest annähernd senkrecht abstehenden biegeelastischen Schenkeln (220). Die zumindest bereichsweise annähernd parallel zueinander ausgerichteten Schenkel (220) sind an ihren freien Enden jeweils um 90 Winkelgrade nach innen abgewinkelt, um dort jeweils ein Halteelement (221) zu bilden. Die Halteelemente (221), die z.B. 1,5 bis 3 Millimeter lang sind, ragen aufeinander zu. Sie bilden eine Ebene, die parallel zur Stirnplatte (210) ausgerichtet ist.

Anstelle des hakenförmigen Halteelements (221) kann in jedem Schenkel (220) eine Ausnehmung vorgesehen sein, in die der Zylinder der Zylinder-Kolben-Einheit (100) mittels jeweils eines Zapfens eingehängt werden kann.

An den Übergangsstellen zwischen der Stirnplatte (210) und den Schenkeln (220) sind nach Figur 5 jeweils zwei Versteifungssicken (211) eingedrückt. Die Versteifungssicken (211) ragen soweit in die Stirnplatte (210) hinein, dass sie zudem die letzte Windung der Schraubendruckfeder (50) auf der Stirnplatte (210) zentrieren.

Im mittleren Bereich ist jeder Schenkel (220) z- oder s-förmig gebogen, wobei die doppelwinkelförmige Biegung spiegelsymmetrisch zur Mittellinie (5) ausgeführt ist, vgl. Figur 4. In dieser Figur ist der Blechstreifen (201) im entspannten Zustand dargestellt. Der mittlere Bereich ist hierbei eine Zone, die sich nach Figur 4 oberhalb und unterhalb der Mitte des Gehäuses (200) um ca. ein Viertel der Gehäusegesamtlänge erstreckt.

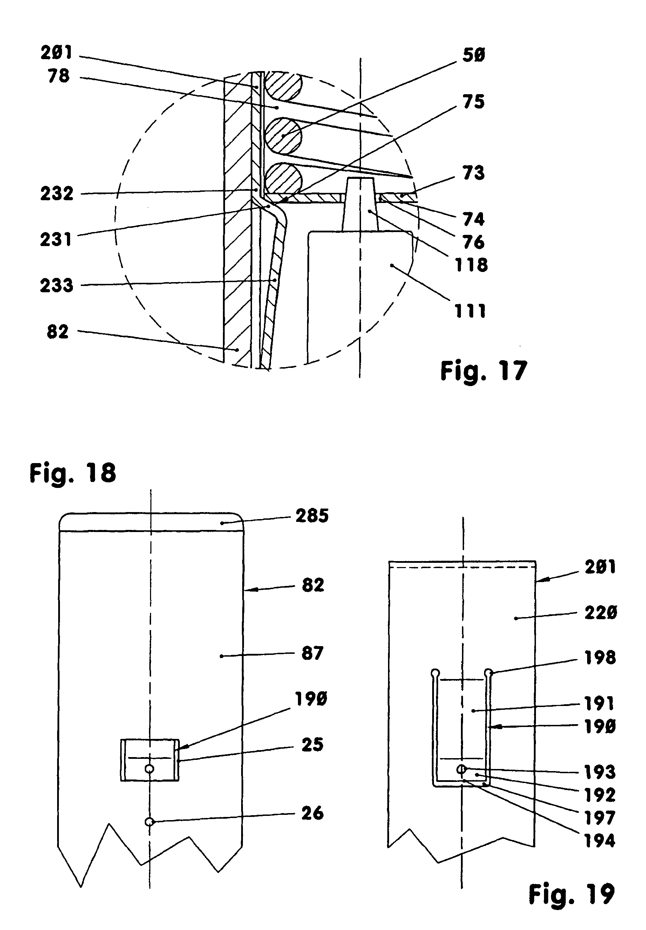

Jeder Schenkel (220) besteht aus einem vorderen Halteabschnitt (233), einem mittleren Abstützabschnitt (231) und einem hinteren Anlageabschnitt (232). Der Halteabschnitt (233) ist überwiegend gerade ausgeführt und verläuft nach Figur 4 parallel zur Mittellinie (5). An ihn schließt sich der relativ kurze Abstützabschnitt (231) an. Der Abstützabschnitt (231), er misst in Blechstreifenlängsrichtung ca. 1,5 bis 3 Millimeter, vgl. Figur 17, schließt mit dem Halteabschnitt (233) z.B. einen Winkel von 112 bis 118 Winkelgraden ein. Gegenüber der Vertikalen ist er um 65 Winkelgrade geneigt.

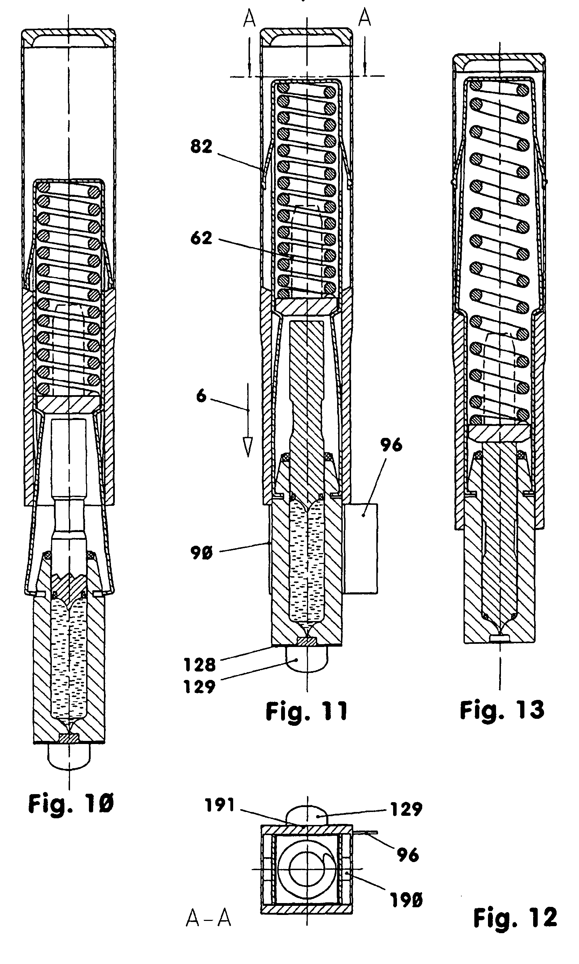

Auf dem Abstützabschnitt (231) liegt der Kolbenbetätigungsstempel (60) bei nicht ausgelöstem Einweginjektor auf, vgl. Figur 1 und 10. Er ist somit auf Zug belastet.

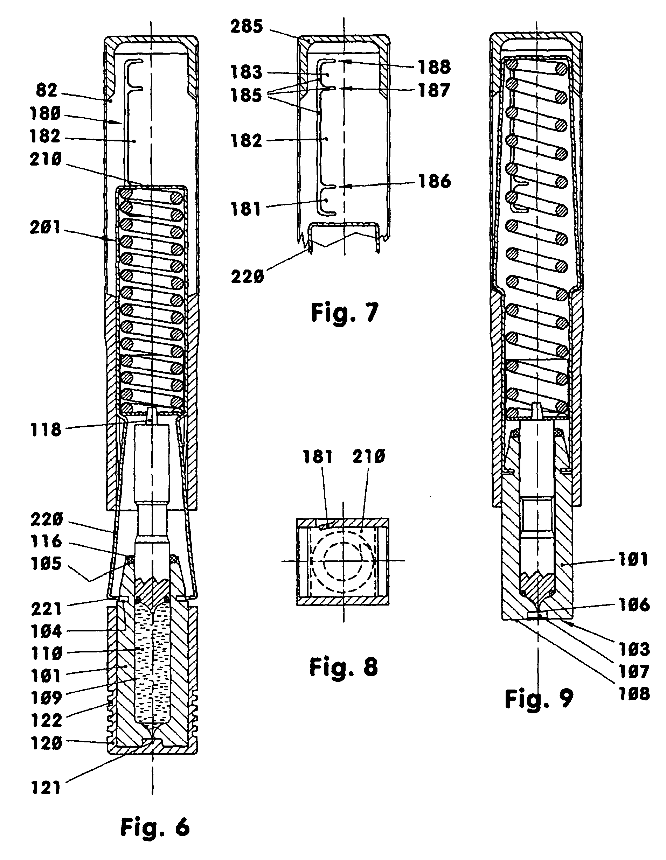

An den Abstützabschnitt (231) schließt sich der Anlageabschnitt (232) an. Er erstreckt sich bis zur Stirnplatte (210). Der Anlageabschnitt (232) schließt mit dem Abstützabschnitt (231) - nach Figur 4 - einen Winkel von 113 ± 3 Winkelgraden ein. Dabei liegt er nach den Figuren 1 und 6 großflächig am Auslöseelement (82) an. Beispielsweise hat das Auslöseelement (82) in der unteren Kontaktzone (86), in der sich bei gespanntem Federelement (50) der Anlageabschnitt (232) großflächig abstützt, eine keramische Panzerung.

Nach den Figuren 1, 10, 11 und 17 liegt auf den Abstützabschnitten (231) der Schenkel (220) der Kolbenbetätigungsstempel (60) auf. Letzterer ist hier ein u-förmig gebogener Blechstreifen, der aus einem Mittelteil, dem Stempelteller (73) und zwei Führungsschenkeln (78) besteht. Der Stempelteller (73) ist parallel zur Stirnplatte (210) orientiert. Die Führungsschenkel (78) stehen rechtwinkelig nach oben ab. Zwischen den Führungsschenkeln (78) sitzt die Schraubendruckfeder (50). Ggf. sind die Führungsschenkel (78) gegenüber dem Stempelteller (73) mit Versteifungssicken ausgestattet, vgl. hierzu die Versteifungssicken (211) des Blechteils (201) aus Figur 5.

Nach Figur 17 hat der Stempelteller (73) in dem Bereich, in dem er an dem jeweiligen Abstützabschnitt (231) des Schenkels (220) anliegt, z.B. eine 25°-Fase (75) zur Sicherstellung einer großflächigen Anlage.

Gemäß Figur 1 hat der Kolbenbetätigungsstempel (60) eine Breite, die geringfügig - also ca. 0,1 bis 0,3 Millimeter - kleiner ist als der reguläre Abstand der beiden Schenkel (220). Demnach wird der Kolbenbetätigungsstempel (60) seitlich an den Schenkeln (220) geführt. In Figur 2 ist zu erkennen, dass die Führungsschenkel (78) des Kolbenbetätigungsstempels (60) an der Innenwandung (89) des Auslöseelements (82) mit Spiel geführt anliegen.

Der Stempelteller (73) hat u.a. nach den Figuren 1 bis 3 und 17 eine zentrale Bohrung (76), um den Kolben (111) der Zylinder-Kolben-Einheit (100) rückwärtig zusätzlich zu führen.

Die beiden auf Zug belasteten Schenkel (220) halten den Kolbenbetätigungsstempel (60) an dessen Stempelteller (73) in seiner vorgespannten Lage, vgl. Figur 1 und 17. Dazu stützen sich die Schenkel (220) mit ihren Abstützabschnitten (231) an der unteren 25°-Fase (75) des Stempeltellers (73) ab. Die Größe der jeweiligen Kontaktfläche zwischen dem einzelnen Abstützabschnitt (231) und der entsprechenden 25°-Fase (75) liegt im Bereich von 10 bis 30 mm².

Das aus Blech gefertigte Gehäuse (200) ist großteils von einem Auslöseelement (82) umgeben, in dem es gleitfähig sitzt. Das Auslöseelement (82) ist hier ein mit einem Deckel (285) rückwärtig verschlossenes vierkantartiges Rohr, das Teil einer Auslöseeinheit (80) ist. Das aus Kunststoff, z.B. einem Polyamid gefertigte Rohr (82) hat einen vorderen (21) und einen hinteren Bereich (22).

Der vordere Bereich (21), der sich ca. auf das vordere Drittel des Auslöseelements (82) bezieht, hat die Form eines Vierkantrohres mit quadratischem Ringquerschnitt. Die vier Seitenwandungen (87, 88) haben in diesem Bereich (21) jeweils die gleiche Wandstärke von 1,5 bis 2,5 Millimeter.

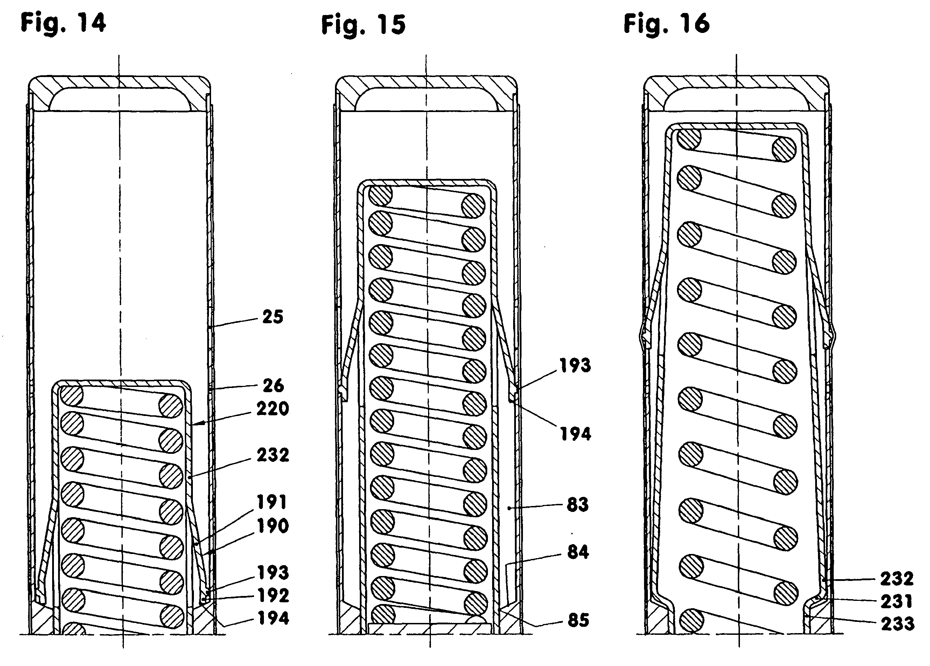

Der hintere Bereich (22) hat einen rechteckigen Ringquerschnitt, vgl. Figur 3, wobei die Seitenwandung (88) um ca. 5 Prozent breiter ist als die Seitenwandung (87). In der Seitenwandung (87) ist bereichsweise eine Längsnut (83) angeordnet, die sich bis zum hinteren Ende des Auslöserohres (82) erstreckt. Im Bereich der Längsnut (83) ist die wandstärke der Seitenwandung (87) auf z.B. 0,5 Millimeter reduziert. Die Längsnut (83) endet vorn in einer z.B. planen Rücksprungflanke (84), die gegenüber der Innenwandung (89) um ca. 75 Winkelgrade geneigt ist, vgl. auch Figur 15. Die Neigung hat dieselbe Orientierung wie die Neigung der Abstützabschnitte (231) der Schenkel (220) des Blechstreifens (201).

Die Längsnuten (83) nehmen bei einem ausgelösten Injektor jeweils den Anlageabschnitt (232) und den Abstützabschnitt (231) des einzelnen Schenkels (220) größtenteils auf, vgl. Figuren 9 13, und 16.

Im hinteren Bereich des Auslöserohres (82) sind nach Figur 2 in der rechten Seitenwandung (88) drei wenige zehntel Millimeter nach innen ragende, elastische Rastlaschen (181-183) angeordnet, vgl. auch Figuren 6 bis 8. Die Rastlaschen (181-183) haben z.B. jeweils eine rechteckige Form. Ihre Wandstärke entspricht ca. 50% der Wandstärke der Seitenwandung (88). Sie grenzen sich an drei Seiten gegenüber der Wandung des Auslöserohres (82) bzw. gegenüber der nächstgelegenen Rastlasche über Spalte (185) ab. Die Spalte (185) hat eine Breite von z.B. 0,5 Millimeter. Die Breite entspricht der Wandstärke der Stirnplatte (210). An den Stellen, an denen jeweils zwei Spalten (185) rechtwinkelig aneinander stoßen, sind die Rastlaschen (181-183) abgerundet.

Die außermittig angeordneten, am Auslöserohr angeformten Rastlaschen (181-183), sichern die Position des Blechstreifens (201) an drei Stellen (186-188). Sie ragen dazu mehrere zehntel Millimeter in den Innenraum (29) des Auslöseelements (82) hinein. Die erste Stelle (186) ist der Spalt zwischen der vorderen (181) und der mittleren Rastlasche (182). Im dortigen horizontalen Spalt ist die Stirnplatte (210) eingerastet, vgl. Figur 6, wenn der Blechstreifen (201) mit der zwischen dem Kolbenbetätigungsstempel (60) und der Stirnplatte (210) eingespannten Schraubendruckfeder (50) zur weiteren Zwischenlagerung montiert ist.

Die zweite Stelle (187) ist der Spalt zwischen der mittleren (182) und der hinteren Rastlasche (183). Nach den Figuren 1 und 2 sitzt hier die Stirnplatte (210) bei einem fertig montierten, nicht ausgelösten Einweginjektor. Durch das Einrasten der Stirnplatte (210) in diesen Spalt wird ein Herausziehen des Gehäuses (200) aus dem Auslöserohr (82) - nach dem Abziehen der Schutzkappe (120) - verhindert. Die dritte Stelle (188) ist der Spalt oberhalb der hinteren Rastlasche (183). In dieser Position verharrt der Blechstreifen (201) nach dem Auslösen des Injektors, vgl. Figur 9. Dort ist er gegen eine unerwünschte Demontage des dann ge- bzw. verbrauchten Injektors gesichert.

Ggf. sind die jeweils oberen Ecken der Rastlaschen (181-183) - also die, die dem Deckel (285) zugewandt sind - scharfkantig ausgebildet, so dass der Blechstreifen (201) nur in das Auslöserohr (82) hineingeschoben werden kann. Eine Bewegung in die entgegengesetzte Richtung ist dann unmöglich.

Bei der bisher gezeigten Variante sind alle Rastelemente (181-183) am Auslöseelement (82) angeordnet. Sie fixieren teilweise temporär, teilweise dauerhaft die Position der Stirnplatte (210) gegenüber dem Auslöseelement (82). Es ist auch denkbar, die Rastelemente (181-183) durch mindestens ein am Gehäuse (200) angeordnetes Rastelement zu ersetzen. Letzteres greift dann z.B. in entsprechende Aussparungen des Auslöseelements (82) ein, um vergleichbare Rastpositionen zu realisieren. In den Figuren 10 bis 16, 18 und 19 ist eine derartige Variante gezeigt.

Dazu ist z.B. im hinteren Viertel eines jeden Schenkels (220) eine z.B. 6 Millimeter breite Rastzunge (190) angeordnet, vgl. Figur 19. Die Rastzunge (190) entsteht durch das Herausarbeiten eines u-förmigen, z.B. 0,2 bis 0,5 Millimeter breiten Spaltes (197). Der Spalt (197) endet im jeweils hinteren Bereich der Schenkel (220), also in der Nähe der Stirnplatte (210) in Bohrungen (198) zur Minimierung der dortigen Kerbspannungen. Im Gegensatz zu den im Wesentlichen planen Anlageabschnitt (232) des Schenkels (220) ist die Rastzunge (190) mehrfach gebogen, vgl. auch Figuren 14 bis 16. Die Rastzunge (190) besteht aus einem nach außen federnden Biegeabschnitt (191) und einem Stützabschnitt (192). Letzterer weist ca. mittig eine Rastnoppe (193) auf.

In den Figuren 14 und 10 liegt jeweils die Rastzunge (190) mit ihrer Abstützstirnfläche (194) auf der Rücksprungflanke (84) der Längsnut (83) des Auslöseelements (82) auf. In dieser Position dient die Rastzunge (190) als Transportsperre. Dies entspricht der ersten Stelle (186) des Rastgesperres (180) aus den Figuren 6 und 7.

Die Figuren 15 und 11 zeigen den Injektor im Handelszustand. Die Rastnoppen (193) der Rastzunge (190) sind in die Rastbohrungen (26) des Auslöseelements (82) eingerastet.

Nach dem Auslösen des Injektors rastet die Rastzunge (190) mit ihrem Stützabschnitt (192) in das Fenster (25) des Auslöseelements (82). Der Biegeabschnitt (191) liegt hierbei an der hinteren Kante des Fensters (25) auf, während sich die Stützstirnfläche (194) an der vorderen Kante des Fensters (25) abstützt. Aus dieser Rastposition kann der Blechstreifen (201) nach vorn aus dem Auslöserohr (82) nicht mehr herausgezogen werden.

Die Rastausnehmungen (25, 26) und die Spalte (185, 197) sind beim fertig montierten Einweginjektor z.B. durch eine dauerhaft aufgeklebte oder aufgeschrumpfte, z.B. beschriftete ggf. elastische Folie staubdicht abgedeckt.

Am hinteren Ende ist das Auslöserohr (82) mit einem Deckel (285) verschlossen. Der Deckel (285) ist beispielsweise mit dem Auslöseelement (82) verklebt, verschweißt, verrastet oder verstaucht. Ggf. ist der Deckel am Auslöseelement (82) auch angeformt. Der Deckel (285) hat zwei einander gegenüber liegende Deckelzungen (286), die jeweils den Querschnitt der Längsnuten (83) der Seitenwandungen (87) im hinteren Auslöserohrbereich ausfüllen. Die Deckelzungen (286), die an ihren freien Enden zum Innenraum (29) hin angefast sind, ragen so weit in das Auslöserohr (82) hinein, dass sie nach Figur 1 den Blechstreifen (201) im Bereich der Stirnplatte (210) seitlich mit geringem Spiel abstützen können, sofern sich der Injektor im Handelszustand befindet.

Die Zylinder-Kolben-Einheit (100) besteht im Ausführungsbeispiel aus einem, mit einer Injektionslösung (1) oder einem Lösemittel, z.B. Wasser für Injektionszwecke, befüllten, transparenten Zylinder (101), in dem nach Figur 1 ein Kolben (111) in seiner hinteren Position sitzt.

Der Zylinder (101) ist z.B. ein dickwandiger Topf. Die Zylinderbohrung ist beispielsweise zylindrisch oder kegelstumpfmantelförmig ausgeführt. Im Zentrum der Bohrung, deren Zylinderboden der Kontur der vorderen Stirnseite des Kolbens (111) zumindest annähernd angepasst ist, befindet sich z.B. eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101), vgl. Figur 9. Ggf. können im Boden des Zylinders (101) auch zwei oder mehr düsenartige Bohrungen (106) angeordnet sein.

Um die Ausnehmung (107) herum klebt fest haftend auf der Stirnfläche (103) ein Klebering (108). Letzterer deckt nahezu die gesamte Stirnfläche (103) ab.

Die räumliche Außenkontur des Zylinders (101) ist im Ausführungsbeispiel z.B. quaderförmig gestaltet. Sie kann jedoch auch zylindrisch sein. Der Querschnitt der Außenkontur - er ist quer zur Mittellinie (5) orientiert - ist im mittleren Zylinderbereich eine quadratische Fläche mit zentraler Bohrung. Der Querschnitt ist so dimensioniert, dass der Zylinder (101) mit geringem Spiel im Innenraum (29) des Auslöserohres (82) gleitet.

Der Zylinder (101) hat in seiner Außenkontur im oberen, dem Auslöserohr (82) zugewandten Viertel eine z.B. umlaufende Haltekerbe (104) mit einem z.B. rechteckigen Kerbquerschnitt. In die Haltekerbe (104) greifen - zum Fixieren des Zylinders (101) im Injektor - die hakenförmigen Halteelemente (221) der Schenkel (220) ein. Oberhalb der Haltekerbe (104) verjüngt sich der Zylinder (101) pyramidenstumpfförmig. Der von gegenüberliegenden pyramidalen Flächen eingeschlossene Winkel beträgt z.B. 20 bis 30 Winkelgrade. Die Haltekerbe (104) kann ggf. auch nur aus zwei einander gegenüber liegenden Einzelkerben bestehen.

Der Zylinder (101) hat eine Zylinderinnenwandung (109), die im Bereich der hinteren Zylinderstirnfläche in einer Ringnut (105) zur Aufnahme eines Dichtelements (116) endet.

Der im Zylinder (101) sitzende Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Der Kolben (111) hat in seinem mittleren Bereich eine Taille und an seiner Rückseite z.B. einen zentralen, kegelstumpfförmigen Zapfen (118), der in die Bohrung (76) des Stempeltellers (73) mit Spiel hineinragt.

Der Kolben (111) und das Dichtelement (116) schließen den befüllten Zylinderinnenraum (110) steril ab.

Die zylindrische Ausnehmung (107) der bodenseitigen Stirnfläche (103) des Zylinders (101) ist nach Figur 11 beispielsweise mit einer Schutzfolie (128) verschlossen. Die Schutzfolie (128) klebt über einen Klebering (108) an der Stirnfläche (103). Sie hat seitlich eine Abziehlasche (129). Im mittleren Bereich der Schutzfolie (128) befindet sich ein elastischer, an der Schutzfolie (128) fest haftender Stopfen, der den Hohlraum der Ausnehmung (107) dichtend ausfüllt.

Alternativ hierzu ist u.a. in den Figuren 1 und 2 eine topfförmige Schutzkappe (120) von unten her auf den Zylinder (101) aufgesteckt. Die einteilige Schutzkappe (120), die geometrisch im Prinzip aus fünf ebenen Wandungen besteht, umschließt den Zylinder (101) seitlich mit geringem Spiel. Sie hat im Ausführungsbeispiel nach den Figuren 1-9 den gleichen Vierkantrohrquerschnitt wie der vordere Bereich (21) des Auslöserohres (82). Die obere, z.B. plane Stirnfläche der Schutzkappe (120) kontaktiert die vordere Stirnfläche des vierkantförmigen Auslöseelements (82). Die Außenwandung der Schutzkappe (120) weist eine Profilierung oder Struktur auf, um das Abziehen vom Zylinder (101) zu erleichtern. Im Ausführungsbeispiel wird als Profilierung ein Rillenprofil (122) verwendet.

Der Boden der Schutzkappe (120) weist einen Stopfen (121) auf, der dichtend in die Ausnehmung (107) des Zylinders (101) hineinragt. Die Schutzkappe (120) haftet am Zylinder (101) über den Klebering (108). Letzterer hat gegenüber dem Zylinder (101) eine wesentlich höhere Haftkraft als gegenüber dem Boden der Schutzkappe (120). Um die Haftkraftdifferenz zusätzlich sicherzustellen, ist ggf. der Boden mit einem Profil oder Absatz versehen, so dass die Kontaktfläche gegenüber dem Klebering (108) kleiner ist als die Kontaktfläche zwischen dem Klebering (108) und der zylinderseitigen Stirnfläche (103).

Zwischen dem Stempelteller (73) und der Stirnplatte (210) des Blechstreifens (201) sitzt vorgespannt die Schraubendruckfeder (50). Die Federkraft wird über den Stempelteller (73) auf die Schenkel (220) übertragen. Aufgrund der Neigung der Fase (75) des Stempeltellers (73) werden die Schenkel (220) keilgetriebeartig radial nach außen gedrängt, vgl. Figur 17. Die Fasen (75) kontaktieren die geneigten Abstützabschnitte (231) der Schenkel (220). Die Anlageabschnitte (232) liegen zumindest nahezu plan an der Innenwandung des Auslöserohres (82) an. Das Auslöserohr (82) stützt somit die keilgetriebebedingte Querkraft dauerhaft ab.

Nach den Figuren 1 und 2 berühren sich das vierkantförmige Auslöseelement (82) und die Schutzkappe (120) an ihren Stirnseiten. Als Originalitätsverschluss ist dieser Bereich zusätzlich mit einer Banderole (90) als Sicherungselement umgeben. Die abreiß- oder auftrennbare Banderole (90) ist z.B. ein mit einem Klebstoff einseitig beschichteter Papier- oder Folienstreifen. Der Folienstreifen umgibt z.B. einlagig einmal den Verbund aus Auslöseelement (82) und Schutzkappe (120). Er verklebt die Teile (82) und (120) temporär. Zum Entsichern des Injektors bzw. zum Entfernen der Schutzkappe (120) - bei der Vorbereitung zur Nutzung des Injektors - wird die Banderole (90) abgezogen oder so aufgetrennt, dass die Klebeverbindung zwischen dem Auslöseelement (82) und der Schutzkappe (120) aufgehoben ist. Im Ausführungsbeispiel wird dazu die im Bereich des Auslöseelements (82) liegende Abreißfahne (96) ergriffen und damit die Banderole (90) z.B. bereichsweise abgewickelt. Hierbei reißt die Banderole (90) an einer definierten, z.B. geradlinigen Sollbruchstelle (93) auf, die genau im Bereich der Stirnseiten liegt. Folglich wird - beim Entsichern - nur der auf dem Auslöseelement (82) anliegende Teil (91) der Banderole (90) entfernt.

Die Figuren 6 und 7 zeigen den Injektor bei einem Montagezwischenschritt. Bei der Montage wird zunächst die Schraubendruckfeder (50) mit dem Kolbenbetätigungsstempel (60) und dem Blechstreifen (201) zusammengesteckt. Dazu wird die Schraubendruckfeder (50) in den fertig umgeformten Blechstreifen (201) so eingelegt, dass ein Federende an der Stirnplatte (210) zur Anlage kommt. Auf das andere Federende wird der bügelartige Kolbenbetätigungsstempel (60) aufgeschoben. Nun wird unter Zuhilfenahme einer die Schraubendruckfeder (50) außen oder innen führenden Montagevorrichtung der Blechstreifen (201) zwischen der Stirnplatte (210) und dem Kolbenbetätigungsstempel (60) so weit - entgegen der Federwirkung - zusammengeschoben, dass die Fasen (75) der Stirnseite (74) hinter den Abstützabschnitten (231) zur Anlage kommen, vgl. Figur 17. Hierbei erleichtern die an dem Kolbenbetätigungsstempel (60) seitlich anliegenden Anlageabschnitte (232) den Montagevorgang.

Nun wird die Kombination aus der gespannten Feder (50), dem Blechstreifen (201) und dem Kolbenbetätigungsstempel (60) - immer noch eingespannt in der Montagevorrichtung - von unten her in das Auslöserohr (82) eingeschoben. Der Einschiebevorgang ist beendet, wenn die Stirnplatte (210) in den zwischen den Rastlaschen (181) und (182) gelegenen Spalt (185) einrastet. In dieser Position (186), vgl. Figur 6, ragen die freien Enden der Schenkel (220) unten aus dem Auslöserohr (82) heraus.

In einem weiteren Montageschritt wird die befüllte Zylinder-Kolben-Einheit (100), mit dem Führungszapfen (118) des Kolbens (111) voraus, in das Auslöserohr (82) so eingesteckt, dass zum einen der Führungszapfen (118) in die Bohrung (76) des Kolbenbetätigungsstempels (60) hineinragt und zum anderen die Halteelemente (221) der Schenkel (220) in die Haltekerbe (104) des Zylinders (101) hineingreifen. Ausgehend von dieser Position wird das Auslöserohr (82) weiter über den Blechstreifen (201) geschoben, bis die Stirnplatte (210) in den zwischen den Rastlaschen (182) und (183) gelegenen Spalt (185) einrastet. Hierbei greifen die Halteelemente (221) fest in die Haltekerbe (104) ein und fixieren so die Zylinder-Kolben-Einheit (100) im Auslöserohr (82). Gegenüber dem in Figur 1 dargestellten Montageschritt fehlt nur noch das Anbringen des Originalitätsverschlusses (90) und das Überkleben bzw. Abdecken der Längsnuten (83) und der Spalte (185) mittels einer beschrifteten Folie.

Die Figuren 10 bis 16, 18 und 19 zeigen eine gegenüber den Figuren 1 bis 9 teilweise abweichende Variante. Sie unterscheidet sich u.a. in sieben Punkten. Erstens hat der Blechstreifen (201) zur seitlichen Zentrierung zwischen den Seitenwandungen (87) des Auslöserohres (82) pro Schenkel (220) mindestens eine Rastzunge (190). Zweitens ist der Kolbenbetätigungsstempel (60) nur eine Vierkantplatte ohne Bohrung, die an ihrer unteren Stirnseite (74) zwei oder vier Fasen (75) aufweist. Ggf. ist an der oberen Stirnseite der Vierkantplatte ein Führungszapfen (62) - hier gestrichelt eingezeichnet - befestigt oder angeformt. Drittens hat der Kolben (111) an seiner hinteren Stirnseite keinen Führungszapfen. Viertens hat das Auslöseelement (82) anstelle der Rastlaschen (181-183), vgl. Figur 7, nur die Rastausnehmungen (25, 26). Fünftens hat das Auslöseelement (82) einen Deckel (285) ohne die Deckelzungen (286) nach Figur 1. Sechstens weist der Zylinder (101) anstelle der Schutzkappe (120), vgl. Figur 1, nur eine Schutzfolie (128) auf, vgl. Figur 10 und 11. Siebtens ist die Banderole (90) nur um den Zylinder (101) gewickelt. Allerdings hat die Folie der Banderole (90) eine so große Wandstärke, dass sie ein Verschieben des Auslöseelements (82) in Auslöserichtung (6) sicher blockiert.

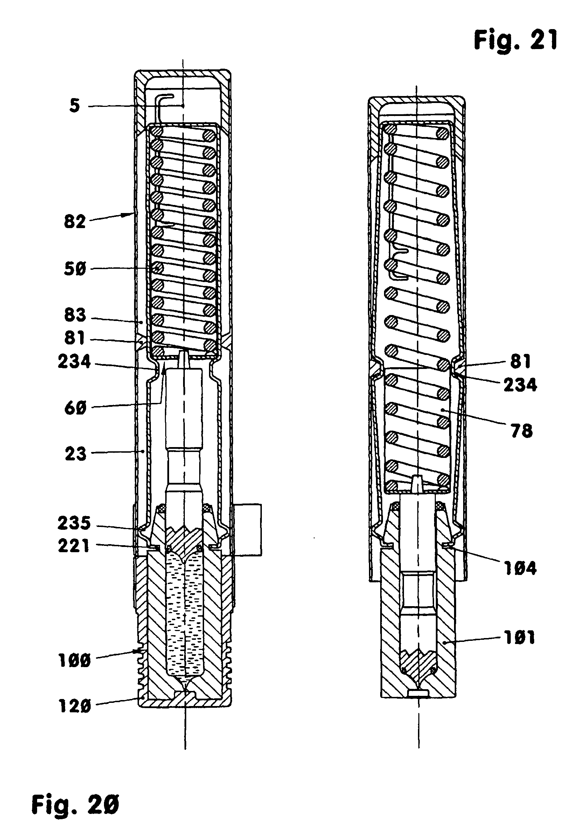

Die Figuren 20 und 21 zeigen einen Injektor, dessen Blechstreifen (201) mit einer Rasteinstülpung (234) und einer Führungsausstülpung (235) ausgestattet ist. Dieser Blechstreifen (201) gehört zu einem Auslöserohr (82), das pro Seitenwandung (87) eine hintere (83) und eine vordere Längsnut (23) aufweist. Die Längsnuten (83, 23) sind durch einen mehrere Millimeter breiten Steg (81), der ca. mittig im Auslöserohr angeordnet ist, voneinander getrennt.

Die Rasteinstülpung (234) des Blechstreifens (201), deren hinterer Bereich der Abstützabschnitt (231) ist, ist so geformt, dass sie bei ausgelöstem Injektor den Steg (81) mit Spiel umgibt, vgl. Figur 21.

Die wellenförmige Führungsausstülpung (235) befindet sich in der Nähe der Halteelemente (221). Sie hat die Aufgabe, die vorderen Enden der Schenkel (220) mit ihren Halteelementen (221) so zu stützen, dass die Halteelemente (221) in jedem Betriebszustand des Injektors sicher in die Haltekerben (104) des Zylinders (101) eingreifen.

Zur Vorbereitung der Benutzung der in den Figuren dargestellten Einweginjektoren wird der einzelne Injektor zunächst durch das Ablösen der Abreißfahne (96) entsichert. Anschließend wird die Schutzkappe (120) oder die Schutzfolie (128) von der Zylinder-Kolben-Einheit (100) abgezogen. Nun wird der Injektor mit dem Klebering (108) voraus - auf der desinfizierte Injektionsstelle positioniert. Dabei wird der Einweginjektor am Auslöserohr (82) in der Faust gehalten. Der Daumen der haltenden Hand liegt beispielsweise auf dem Deckel (285) auf, z.B. wie beim Halten eines Kugelschreibers.

Nun wird das Auslöserohr (82) in Richtung der Zylinder-Kolben-Einheit (100) verschoben. Bei diesem Vorgang gleitet das Auslöseelement (82) auf dem Blechstreifen (201) linear nach unten, also in Richtung der Injektionsstelle. Die Anlageabschnitte (232) der Schenkel (220) rutschen über die Kante (85) und springen unter der Kraft des Federelements (50) entsichernd in Querrichtung nach außen in die Längsnuten (83). Die Abstützabschnitte (231) geben den Kolbenbetätigungsstempel (60) frei. Dieser schnellt ungehindert nach unten. Dabei schlägt die Stirnseite (74) des Stempeltellers (73) auf die Stirnseite des bisher einige zehntel Millimeter oder wenige Millimeter entfernt gelegenen Kolbens (111). Der Kolben (111) drückt die Injektionslösung bzw. das Medikament (1) z.B. anfangs mit 300 x 10⁵ Pa durch die Düse (106), bis der Zylinder (101) entleert ist, vgl. Figur 9. Mit der Abgabe der Injektionslösung (1) ist der Injektionsvorgang beendet.

Die Ausführungsbeispiele zeigen Injektoren, deren gehäuseseitige Schenkel (220) jeweils paarweise zumindest annähernd parallel - eine Winkelabweichung von ± 2 Winkelgraden ist zulässig - zueinander ausgerichtet sind. Die Schenkel (220) liegen hierbei in parallelen Ebenen, wobei die Ebenen - im Injektorquerschnitt gesehen - die einander gegenüberliegenden Seiten eines Rechtecks bilden. Die Ebene des Injektorquerschnitts liegt normal - also senkrecht - zur Mittellinie (5). Diese Seiten können auch zu einer Raute, einem Parallelogramm, einem Trapez oder einem schiefwinkeligen Viereck gehören.

Zudem sind die Schenkel (220) jeweils paarweise gleich lang und die Abstützabschnitte (231) liegen sich auf der gleichen Höhe gegenüber, vgl. Figuren 1, 4, 9 u.s.w. Das ist nicht zwingend notwendig. So können z.B. die Abstützabschnitte (231) auf unterschiedlichen Höhen liegen, wenn entsprechend die Auflageflächen des Kolbenbetätigungsstempels (60) und die Rücksprungflächen (84) versetzt positioniert werden.

Anstelle des aus einem einzigen Blechstreifen (201) bestehenden Gehäuses (200) können auch zwei vergleichbare Blechstreifen miteinander über Kreuz kombiniert werden. Die Blechstreifen sind gegenüber der Mittellinie (5) um 90 Winkelgrade zueinander versetzt angeordnet. Auf diese Weise wird z.B. der Stempelteller (73) an vier Seiten von den Abstützabschnitten (231) umgriffen. Dies gilt ggf. auch für die Halterung des Zylinders (101).

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil

- 21: vorderer Bereich von (82)
- 22: hinterer Bereich von (82)
- 23: Längsnut, vorn
- 25: Rastfenster, Durchbruch, Rastausnehmung
- 26: Rastbohrung, Rastausnehmung
- 29: Innenraum von (82)

- 50: Federelement, Schraubendruckfeder, Federenergiespeicher

- 60: Kolbenbetätigungsstempel
- 62: Führungszapfen

- 73: Stempelteller
- 74: Stirnseite, unten
- 75: Fase, 25°-Fase
- 76: Bohrung
- 78: Führungsschenkel

- 80: Auslöseeinheit
- 81: Steg
- 82: Auslöseelement, Auslöserohr, Rohr
- 83: Längsnuten, Ausnehmungen
- 84: Rücksprungflanke
- 85: Kante, scharfkantig
- 86: Kontaktzone
- 87: Seitenwandung mit Längsnut (83)
- 88: Seitenwandung ohne Längsnut (83)
- 89: Innenwandung

- 90: Originalitätsverschluss, Banderole, Sicherungselement
- 91: hinterer Banderolenabschnitt, an (82); Teil
- 92: vorderer Banderolenabschnitt, an (120)
- 93: Sollbruchstelle, Perforation
- 96: Abreißfahne

- 100: Zylinder-Kolben-Einheit
- 101: Zylinder
- 103: Stirnfläche
- 104: Haltekerbe
- 105: Ringnut
- 106: Bohrung, Düse
- 107: Ausnehmung in der Stirnfläche
- 108: Klebering
- 109: Zylinderinnenwandung
- 110: Zylinderinnenraum
- 111: Kolben
- 112: Ringnut
- 114: Dichtring, Dichtung
- 116: Dichtelement in (105)
- 118: Führungszapfen

- 120: Schutzkappe
- 121: Stopfen
- 122: Rillenprofil
- 128: Schutzfolie, Klebeversiegelung
- 129: Abziehlasche

- 180: Rastgesperre
- 181: Rastlasche, vorn; Rastelement
- 182: Rastlasche, Mitte; Rastelement
- 183: Rastlasche, hinten; Rastelement

- 185: Spalte
- 186: 1. Stelle
- 187: 2. Stelle
- 188: 3. Stelle

- 190: Rastzunge
- 191: Biegeabschnitt
- 192: Stützabschnitt
- 193: Rastnoppe
- 194: Stützstirnfläche

- 197: Spalt, c-förmig
- 198: Bohrungen

- 200: Gehäuse; Blechteil, dünnwandig
- 201: Blechstreifen; Blechteil

- 210: Stirnplatte
- 211: Sicken, Versteifungssicken

- 220: Schenkel, lang und breit, Biegebalken
- 221: Halteelemente

- 231: Abstützabschnitt
- 232: Anlageabschnitt
- 233: Halteabschnitt
- 234: Rasteinstülpung
- 235: Führungsausstülpung

- 285: Deckel
- 286: Deckelzungen

## Patentansprüche

1. Nadelloser Einmalinjektor mit einem Gehäuse (200), in dem oder an dem - jeweils zumindest bereichsweise - mindestens ein mechanischer Federenergiespeicher (50), mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet sind,
- wobei der Kolbenbetätigungsstempel (60) zwischen dem Federenergiespeicher (50) und dem Kolben (111) der Zylinder-Kolben-Einheit (100) positioniert ist,
- wobei der Federenergiespeicher mindestens ein vorgespanntes Federelement (50) umfasst und
- wobei der federbelastete Kolbenbetätigungsstempel (60) am Gehäuse (200) lösbar abgestützt ist,
**dadurch gekennzeichnet,**
- **dass** das Gehäuse (200) aus einem dünnwandigen Blechteil (201) besteht,
- **dass** das Blechteil (201) mindestens zwei Schenkel (220) aufweist,
- **dass** die Schenkel (220) an den freien Enden - als Aufnahme des Zylinders (101) der Zylinder-Kolben-Einheit (100) - jeweils ein abgewinkeltes Halteelement (221), das mit einer Haltekerbe (104) des Zylinders (101) kooperiert, oder jeweils eine Ausnehmung die mit einem Zapfen des Zylinders kooperiert aufweisen,
- **dass** die Schenkel (220) elastische Biegebalken sind, die im mittleren Bereich zur Ausbildung eines Abstützabschnitts (231) für den Kolbenbetätigungsstempel (60) und eines Anlageabschnitts (232) für ein Auslöseelement (82) jeweils z- oder s-förmig abgebogen sind,
- **dass** die zwischen einem einzelnen Abstützabschnitt (231) und dem Kolbenbetätigungsstempel (60) gelegene Kontaktzone ein den jeweiligen Schenkel (220) nach außen drängendes Keilgetriebepaar darstellt,
wobei das axial bewegliche Auslöseelement (82) die Anlageabschnitte (232) der Schenkel (220) nach innen hält und somit die Abstützabschnitte (231) in der den Kolbenbetätigungsstempel (60) blockierenden Position hält, wobei das Auslöseelement (82) entlang der Längsrichtung (5) des Injektors zu einer Position beweglich ist, in der es das durch das Keilgetriebepaar verursachte radiale nach außen Drängen der Abstützanschnitte (231) und der Anlegabschnitte (232) in Längsnuten (83) des Auslöseelements (82) zulässt, so dass sich der Kolbenbetätigungsstempel (60) unter der Wirkung des Federenergiespeichers (50) bewegen kann.

2. Einmalinjektor gemäß Anspruch 1,
**dadurch gekennzeichnet,**
**dass** das Gehäuse (200) aus einem Blechstreifen (201) besteht und der Blechstreifen (201) zur Ausbildung zweier Schenkel (220) u-förmig gebogen ist.

3. Einmalinjektor gemäß Anspruch 1,
**dadurch gekennzeichnet,**
**dass** der Blechstreifen (201) an den beiden freien Enden - als Auflage für den Zylinder (101) - nach innen abgewinkelte Halteelemente (221) aufweist.

4. Einmalinjektor gemäß Anspruch 1,
**dadurch gekennzeichnet,**
**dass** die Schenkel (220) mindestens fünfmal länger sind als die Schenkelbreite.

5. Einmalinjektor gemäß Anspruch 1,
**dadurch gekennzeichnet,**
**dass** der Abstützabschnitt (231) mit dem das jeweilige Halteelement (221) tragenden Abschnitt (233) einen Winkel von 115 ± 2 Winkelgraden einschließt.

6. Einmalinjektor gemäß Anspruch 1,
**dadurch gekennzeichnet,**
**dass** der Abstützabschnitt (231) mit dem Abschnitt (232), an dem das Federelement (50) bei gespanntem Injektor seitlich anliegt, einen Winkel von 113 ± 2 Winkelgraden einschließt.

7. Einmalinjektor gemäß Anspruch 1,
**dadurch gekennzeichnet,**
**dass** das Blechteil (201) aus einem Federstahl gefertigt ist.

8. Einmalinjektor gemäß Anspruch 1,
**dadurch gekennzeichnet,**
**dass** das Auslöseelement (82) ein das Blechteil (201) umgebendes Auslöserohr ist.

9. Einmalinjektor gemäß Anspruch 1,
**dadurch gekennzeichnet,**
**dass** der Kolbenbetätigungsstempel (60) eine ebene Platte (73) mit rechteckiger Grundfläche ist oder aus einem u-förmig gebogenen Blechstreifen (73, 78) besteht.

10. Einmalinjektor gemäß Anspruch 1,
**dadurch gekennzeichnet,**
**dass** das Auslöseelement (82) in Kombination mit dem Gehäuse (200) und einer an ihm befestigten Abreißbanderole (90) eine Auslöseeinheit (80) bildet.

## Claims

1. Needleless disposable injector with a housing (200) in which or on which are arranged, in each case at least in some areas, at least one mechanical spring energy reservoir (50), at least one cylinder/piston unit (100) that can be filled at least temporarily with active substance, at least one piston-actuating ram (60) and at least one trigger unit (80),
- wherein the piston-actuating ram (60) is positioned between the spring energy reservoir (50) and the piston (111) of the cylinder/ piston unit (100),
- wherein the spring energy reservoir comprises at least one pretensioned spring element (50), and
- wherein the spring-loaded piston-actuating ram (60) is supported on the housing (200) in a releasable manner,
**characterized in that**
- the housing (200) is made from a thin-walled sheet-metal part (201),
- the sheet-metal part (201) has at least two branches (220),
- the branches (220) each have, at the free ends thereof, an angled retaining element (221), which cooperates with a retaining notch (104) of the cylinder (101), or in each case a recess, which cooperates with a pin of the cylinder, as a means of receiving the cylinder (101) of the cylinder/piston unit (100),
- the branches (220) are elastic flexural beams which, in the middle area, are each bent in a Z-shape or S-shape to form a supporting portion (231) for the piston-actuating ram (60) and a bearing portion (232) for a trigger element (82),
- the contact zone situated between an individual supporting portion (231) and the piston-actuating ram (60) represents a wedge gear pairing that forces the respective branch (220) outwards,
wherein the axially movable trigger element (82) holds the bearing portions (232) of the branches (220) inwards and thus holds the supporting portions (231) in the position blocking the piston-actuating ram (60), wherein the trigger element (82) is movable along the longitudinal direction (5) of the injector to a position in which it allows the supporting portions (231) and the bearing portions (232) to be forced radially outwards, as caused by the wedge gear pairing, in longitudinal grooves (83) of the trigger element (82), such that the piston-actuating ram (60) can move under the effect of the spring energy reservoir (50).

2. Disposable injector according to Claim 1,
**characterized in that**
the housing (200) is made from a sheet-metal strip (201), and the sheet-metal strip (201) is bent in a U-shape to form two branches (220).

3. Disposable injector according to Claim 1,
**characterized in that**
the sheet-metal strip (201) has, at both free ends, inwardly angled retaining elements (221) as a bearing for the cylinder (10).

4. Disposable injector according to Claim 1,
**characterized in that**
the branches (220) are at least five times longer than the width of the branches.

5. Disposable injector according to Claim 1,
**characterized in that**
the supporting portion (231) encloses an angle of 115 ± 2 degrees with the portion (233) that carries the respective retaining element (221).

6. Disposable injector according to Claim 1,
**characterized in that**
the supporting portion (231) encloses an angle of 113 ± 2 degrees with the portion (232) on which the spring element (50) laterally bears when the injector is tensioned.

7. Disposable injector according to Claim 1,
**characterized in that**
the sheet-metal part (201) is made from a spring steel.

8. Disposable injector according to Claim 1,
**characterized in that**
the trigger element (82) is a trigger tube surrounding the sheet-metal part (201).

9. Disposable injector according to Claim 1,
**characterized in that**
the piston-actuating ram (60) is a flat plate (73) with a rectangular surface area or is made from a sheet-metal strip (73, 78) bent in a U-shape.

10. Disposable injector according to Claim 1,
**characterized in that**
the trigger element (82), in combination with the housing (200) and with a tear-off banderole (90) secured thereon, forms a trigger unit (80).

## Revendications

1. Injecteur sans aiguille à usage unique doté d'un boîtier (200), dans lequel ou sur lequel - chaque fois au moins localement - au moins un accumulateur d'énergie élastique mécanique (50), au moins une unité cylindre-piston (100) - à remplir au moins partiellement de substance active -, au moins un poussoir d'actionnement de piston (60) et au moins une unité de déclenchement (80) sont disposés,
- dans lequel le poussoir d'actionnement de piston (60) est positionné entre l'accumulateur d'énergie élastique (50) et le piston (111) de l'unité cylindre-piston (100),
- dans lequel l'accumulateur d'énergie élastique comprend au moins un élément de ressort précontraint (50), et
- dans lequel le poussoir d'actionnement de piston à ressort (60) est appuyé de façon amovible sur le boîtier (200),
**caractérisé en ce que**
- le boîtier (200) se compose d'une pièce de tôle mince (201),
- la pièce de tôle (201) présente au moins deux branches (220),
- les branches (220) présentent aux extrémités libres - comme logement du cylindre (101) de l'unité cylindre-piston (100) - respectivement un élément de retenue coudé (221), qui coopère avec une encoche de retenue (104) du cylindre (101), ou respectivement un évidement, qui coopère avec un tourillon du cylindre,
- les branches (220) sont des bras flexibles élastiques, qui sont respectivement pliés en forme de z ou de s dans la région moyenne pour la formation d'une partie d'appui (231) pour le poussoir d'actionnement de piston (60) et d'une partie d'application (232) pour un élément de déclenchement (82),
- la zone de contact située entre une partie d'appui individuelle (231) et le poussoir d'actionnement de piston (60) représente une paire de moulures en coin qui repoussent la branche respective (220) vers l'extérieur,
- dans lequel l'élément de déclenchement (82) mobile axialement retient les parties d'application (232) des branches (220) vers l'intérieur et retient ainsi les parties d'appui (231) dans la position bloquant le poussoir d'actionnement de piston (60),
- dans lequel l'élément de déclenchement (82) est mobile le long de la direction longitudinale (5) de l'injecteur jusqu'à une position, dans laquelle il permet l'écartement radial vers l'extérieur des parties d'appui (231) et des parties d'application (232) provoqué par la paire de moulures en coin dans des rainures longitudinales (83) de l'élément de déclenchement (82), de telle manière que le poussoir d'actionnement de piston (60) puisse se déplacer sous l'action de l'accumulateur d'énergie élastique (50).

2. Injecteur à usage unique selon la revendication 1, **caractérisé en ce que** le boîtier (200) se compose d'une bande de tôle (201) et la bande de tôle (201) est pliée en forme de U pour la formation de deux branches (220).

3. Injecteur à usage unique selon la revendication 1, **caractérisé en ce que** la bande de tôle (201) présente aux deux extrémités libres - comme appui pour le cylindre (101) - des éléments de retenue (221) coudés vers l'intérieur.

4. Injecteur à usage unique selon la revendication 1, **caractérisé en ce que** les branches (220) sont au moins cinq fois plus longues que larges.

5. Injecteur à usage unique selon la revendication 1, **caractérisé en ce que** la partie d'appui (231) forme avec la partie (233) portant l'élément de retenue respectif (221) un angle de 115 ± 2 degrés d'angle.

6. Injecteur à usage unique selon la revendication 1, **caractérisé en ce que** la partie d'appui (231) forme avec la partie (232), sur laquelle l'élément de ressort (50) s'applique latéralement lorsque l'injecteur est tendu, un angle de 113 ± 2 degrés d'angle.

7. Injecteur à usage unique selon la revendication 1, **caractérisé en ce que** la pièce de tôle (201) est fabriquée en un acier à ressort.

8. Injecteur à usage unique selon la revendication 1, **caractérisé en ce que** l'élément de déclenchement (82) est un tube de déclenchement entourant la pièce de tôle (201).

9. Injecteur à usage unique selon la revendication 1, **caractérisé en ce que** le poussoir d'actionnement de piston (60) est une plaque plane (73) avec une face de base rectangulaire ou se compose d'une bande de tôle pliée en forme de U (73, 78).

10. Injecteur à usage unique selon la revendication 1, **caractérisé en ce que** l'élément de déclenchement (82) forme, en combinaison avec le boîtier (200) et une bandelette à arracher (90) fixée à celui-ci, une unité de déclenchement (80).
